# EUROPEAN PATENT APPLICATION

(11) **EP 2 363 397 A1**
(43) Date of publication of application: **07.09.2011**
(21) Application number: 09824767.9
(22) Date of filing: 02.11.2009
(51) Int. Cl.: C07D 401/04, A61K 31/517, A61K 45/00, A61P 13/00, A61P 13/02, A61P 13/08, A61P 13/10, A61P 43/00

(54) **NOVEL USEFUL THERAPEUTIC AGENT FOR LOWER URINARY TRACT SYMPTOM**

(30) Priority: 07.11.2008 JP 2008286419
(71) Applicant: Dainippon Sumitomo Pharma Co., Ltd., Osaka 541-8524 (JP)
(72) Inventor: GOTO, Natsuko, Osaka-shi Osaka 554-0022 (JP); YAMAMOTO, Setsuko, Osaka-shi Osaka 554-0022 (JP)
(74) Representative: Duckworth, Timothy John
(86) International application number: PCT/JP2009/068763
(87) International publication number: WO 2010/053068

(57) **Abstract**

Provided is a therapeutic agent for lower urinary tract symptom consisting of (+)-3-{1-[3-(trifluoromethoxy)benzyl]piperidin-4-yl}-4-phenyl-3,4-dihydro-2(1H)-quinazolinone or a pharmaceutically acceptable salt thereof or a combination of (+)-3-{1-[3-(trifluoromethoxy)benzyl]piperidin-4-yl}-4-phenyl-3,4-dihydro-2(1H)-quinazolinone or a pharmaceutically acceptable salt thereof and a compound selected from the group consisting of α₁-adrenergic receptor blockers, 5-α reductase inhibitors, antiandrogen drugs, β₃-adrenergic receptor agonists, phosphodiesterase V inhibitors, plant preparations, amino acid preparations, herbal medicines and the like. Also provided is a method of using in combination the above-described therapeutic agent and a surgical treatment such as bladder distention or surgery.

## Description

### Technical Field

The present invention relates to a novel useful therapeutic agent for lower urinary tract symptoms.

### Background Art

Lower urinary tract symptoms generally refer to symptoms caused by a wide variety of diseases such as benign prostatic hyperplasia and interstitial cystitis, including urine storage symptoms such as increased daytime frequency, nocturia, urinary urgency, urge urinary incontinence, stress urinary incontinence, and mixed urinary incontinence; voiding symptoms such as slow stream, weak urine streams, and urine stream intermittency; post-micturition symptoms such as feeling of incomplete emptying and post-micturition drips; lower urinary tract pains such as bladder pain and urethral pain; and the like. Also included is overactive bladder syndrome, which an urinary urgency is essential, and, either urge urinary incontinence is present or absent, and accompanied by increased daytime frequency and nocturia (see Non-patent Document 1). The prevalence rate of lower urinary tract symptoms increases with age; it has been reported that about 80% of people 60 years or older have some lower urinary tract symptoms (see Non-patent Document 2).
In benign prostatic hyperplasia, not only urinary retention (voiding symptoms, post-micturition symptoms) due to urethral obstruction occur as lower urinary tract symptoms, but also urine storage symptoms such as increased daytime frequency, nocturia, and urinary urgency are often coincidental. Widely prescribed as therapeutic drugs for benign prostatic hyperplasia, α₁-adrenergic receptor blockers and 5-α reductase inhibitors ameliorate voiding symptoms and post-micturition symptoms by reducing urethral resistance, but they are hardly effective on overactive bladder symptoms, which are characterized by the complaint of urinary urgency. Therefore, in the treatment of these overactive bladder symptoms, in addition to α₁-adrenergic receptor blockers, antimuscarinic drugs are used in combination (see Non-patent Document 3).
Although antimuscarinic drugs such as solifenacin, tolterodine, oxybutynin, propiverine, darifenacin, imidafenacin, trospium, and fesoterodine exhibit a certain effect on lower urinary tract symptoms such as increased daytime frequency and urinary incontinence by blocking the muscarine receptors expressed in the detrusor smooth muscle, they do not have a sufficient ameliorating effect on urinary urgency, which is considered to occur due to activation of the afferent nerve of the urinary bladder (mainly C-fiber).

Meanwhile, a preparation comprising (+)-3-{1-[3-(trifluoromethoxy)benzyl]piperidin-4-yl}-4-phenyl-3,4-dihydro-2(1H)-quinazolinone, that is, the compound represented by the following formula (1):

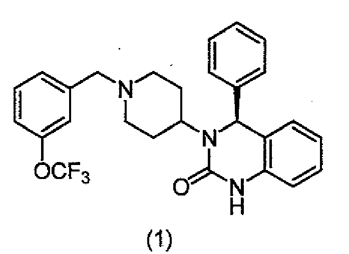

or a pharmaceutically acceptable salt thereof, as an active ingredient, possesses both antimuscarinic action and C-fiber-suppressing action, and is therefore thought to be effective on lower urinary tract symptoms such as increased daytime frequency, nocturia, urinary urgency, urge urinary incontinence, mixed urinary incontinence, bladder pain, and urethral pain when used alone, and to be more effective when used in combination with an α₁-adrenergic receptor blocker, a 5-α reductase inhibitor, an antiandrogen drug, a β₃-adrenergic receptor agonist, a phosphodiesterase V inhibitor, a plant preparation, an amino acid preparation, a herbal medicine, an intravesical instillation or detrusor injection therapy, a serotonin-norepinephrine reuptake inhibitor, a selective serotonin reuptake inhibitor, a selective norepinephrine reuptake inhibitor, a tricyclic antidepressant drug, an α-adrenergic receptor agonist, female hormone replacement therapy, a surgical therapy, a urinary bladder intima protecting agent, an antiallergic drug, an antihistamine drug, a steroid or bladder distention (see Patent Document 1).
Patent Document 1: WO03/016299
Non-patent Document 1: Abrams P., Neurourology and Urodynamics, 21, 167, 2002
Non-patent Document 2: Yukio Honma, Journal of the Japan Neurogenic Bladder Society, 14, 266, 2003
Non-patent Document 3: Chapple CR., European Urology, 49, 651, 2006

### [Summary of the Invention]

### Problems to Be Solved by the Invention

Currently available therapeutic methods for lower urinary tract symptoms such as increased daytime frequency, nocturia, urinary urgency, urge urinary incontinence, stress urinary incontinence, mixed urinary incontinence, bladder pain, and urethral pain include antimuscarinic drugs, α₁-adrenergic receptor blockers, 5-α reductase inhibitors, antiandrogen drugs, β₃-adrenergic receptor agonists, phosphodiesterase V inhibitors, plant preparations, amino acid preparations, herbal medicines, intravesical instillation or detrusor injection therapy, β₂-adrenergic receptor agonists, serotonin-norepinephrine reuptake inhibitors, selective serotonin reuptake inhibitors, selective norepinephrine reuptake inhibitors, tricyclic antidepressant drugs, α-adrenergic receptor agonists, female hormone replacement therapy, surgical therapies, urinary bladder intima protecting agents, antiallergic drugs, antihistamine drugs, steroids, bladder distention and the like; however, in not a few cases, these therapies fail to have a sufficient therapeutic effect when used alone (see Chapple CR., European Urology, 49, 651, 2006).
It is an object of the present invention is to provide a therapeutic agent for lower urinary tract symptom that ensures a sufficient therapeutic effect.

### Means of Solving the Problems

The present inventors conducted extensive investigations to find a novel useful therapeutic method for lower urinary tract symptoms, found that a preparation comprising as an active ingredient the compound represented by the formula (1) or a pharmaceutically acceptable salt thereof, which is a therapeutic drug for overactive bladder syndrome, possesses a suppressing action on the action to raise the membrane potential, which is an indicator of neuron excitement, that is, possesses C-fiber activation-suppressing action, in an investigation with the use of C-fiber-derived small DRG neurons, and that the same preparation possesses a more potent suppressing action when used in combination with an α₁-adrenergic receptor blocker than when used alone, and have completed the present invention.

Accordingly, the present invention relates to the following:
[1] A therapeutic agent for lower urinary tract symptom comprising (+)-3-{1-[3-(trifluoromethoxy)benzyl]piperidin-4-yl}-4-phenyl-3,4-dihydro-2(1H)-quinazolinone or a pharmaceutically acceptable salt thereof as an active ingredient.
[2] A therapeutic agent for lower urinary tract symptom comprising a combination of (+)-3-{1-[3-(trifluoromethoxy)benzyl]piperidin-4-yl}-4-phenyl-3,4-dihydro-2(1H)-quinazolinone or a pharmaceutically acceptable salt thereof, and a drug selected from the group consisting of a therapeutic agent for overactive bladder syndrome, a therapeutic agent for pollakiuria/urinary incontinence, a therapeutic agent for benign prostatic hyperplasia, a therapeutic agent for stress urinary incontinence and a therapeutic agent for interstitial cystitis/painful bladder syndrome.
[3] A therapeutic agent for lower urinary tract symptom comprising a combination of (+)-3-{1-[3-(trifluoromethoxy)benzyl]piperidin-4-yl}-4-phenyl-3,4-dihydro-2(1H)-quinazolinone or a pharmaceutically acceptable salt thereof, and a drug selected from the group consisting of an α₁-adrenergic receptor blocker, a 5-α reductase inhibitor, an antiandrogen drug, a β₃-adrenergic receptor agonist, a phosphodiesterase V inhibitor, a plant preparation, an amino acid preparation, a herbal medicine, an intravesical instillation or detrusor injection therapy, a β₂-adrenergic receptor agonist, a serotonin-norepinephrine reuptake inhibitor, a selective serotonin reuptake inhibitor, a selective norepinephrine reuptake inhibitor, a tricyclic antidepressant drug, an α-adrenergic receptor agonist, a female hormone, a urinary bladder intima protecting agent, an antiallergic drug, an antihistamine drug and a steroid.
[4] The therapeutic agent for lower urinary tract symptom described in [2], wherein the therapeutic agent for benign prostatic hyperplasia is an α₁-adrenergic receptor blocker.
[5] The therapeutic agent for lower urinary tract symptom described in [4], wherein the α₁-adrenergic receptor blocker is selected from the group consisting of naftopidil and tamsulosin.
[6] The therapeutic agent for lower urinary tract symptom described in [2], wherein the therapeutic agent for benign prostatic hyperplasia is a 5-α reductase inhibitor.
[7] The therapeutic agent for lower urinary tract symptom described in [2], wherein the therapeutic agent for benign prostatic hyperplasia is an antiandrogen drug.
[8] The therapeutic agent for lower urinary tract symptom described in [2], wherein the therapeutic agent for overactive bladder syndrome is a β-adrenergic receptor agonist.
[9] The therapeutic agent for lower urinary tract symptom described in [8], wherein the β-adrenergic receptor agonist is a selective β₃-adrenergic receptor agonist.
[10] The therapeutic agent for lower urinary tract symptom described in [2], wherein the therapeutic agent for overactive bladder syndrome or the therapeutic agent for benign prostatic hyperplasia is a phosphodiesterase V inhibitor.
[11] The therapeutic agent for lower urinary tract symptom described in [2], wherein the therapeutic agent for benign prostatic hyperplasia is a plant preparation, an amino acid preparation, or a herbal medicine.
[12] A combination therapy with (+)-3-{1-[3-(trifluoromethoxy)benzyl]piperidin-4-yl}-4-phenyl-3,4-dihydro-2(1H)-quinazolinone or a pharmaceutically acceptable salt thereof, and vesical instillation of resiniferatoxin or capsaicin or urinary bladder wall injection of botulinum toxin, for treating lower urinary tract symptoms.
[13] The therapeutic agent for lower urinary tract symptom described in [2], wherein the therapeutic agent for stress urinary incontinence is a β₂-adrenergic receptor agonist.
[14] The therapeutic agent for lower urinary tract symptom described in [2], wherein the therapeutic agent for stress urinary incontinence is a serotonin-norepinephrine reuptake inhibitor, a selective serotonin reuptake inhibitor, or a selective norepinephrine reuptake inhibitor.
[15] The therapeutic agent for lower urinary tract symptom described in [2], wherein the therapeutic agent for stress urinary incontinence or the therapeutic agent for pollakiuria/urinary incontinence is a tricyclic antidepressant drug.
[16] The therapeutic agent for lower urinary tract symptom described in [2], wherein the therapeutic agent for stress urinary incontinence is an α-adrenergic receptor agonist.
[17] A combination therapy with (+)-3-{1-[3-(trifluoromethoxy)benzyl]piperidin-4-yl}-4-phenyl-3,4-dihydro-2(1H)-quinazolinone or a pharmaceutically acceptable salt thereof, and a surgery for treating benign prostatic hyperplasia, for treating lower urinary tract symptoms.
[18] A combination therapy with (+)-3-{1-[3-(trifluoromethoxy)benzyl]piperidin-4-yl}-4-phenyl-3,4-dihydro-2(1H)-quinazolinone or a pharmaceutically acceptable salt thereof, and a female hormone replacement therapy, for treating lower urinary tract symptoms.
[19] A combination therapy with (+)-3-{1-[3-(trifluoromethoxy)benzyl]piperidin-4-yl}-4-phenyl-3,4-dihydro-2(1H)-quinazolinone or a pharmaceutically acceptable salt thereof, and a surgery for the treatment of stress urinary incontinence, for treating lower urinary tract symptoms.
[20] A combination therapy with (+)-3-{1-[3-(trifluoromethoxy)benzyl]piperidin-4-yl}-4-phenyl-3,4-dihydro-2(1H)-quinazolinone or a pharmaceutically acceptable salt thereof, and an interstitial cystitis/painful bladder syndrome therapy, for treating lower urinary tract symptoms.

### Effect of the Invention

A preparation comprising as an active ingredient the compound represented by the formula (1) or a pharmaceutically acceptable salt thereof, which is a novel therapeutic drug for overactive bladder, possesses both antimuscarinic action and C-fiber-suppressing action, and becomes a novel useful therapeutic agent for lower urinary tract symptom not only when used alone, but also when used in combination with an α₁-adrenergic receptor blocker, a 5-α reductase inhibitor, an antiandrogen drug, a β₃-adrenergic receptor agonist, a phosphodiesterase V inhibitor, a plant preparation, an amino acid preparation, a herbal medicine, an intravesical instillation or detrusor injection therapy, a β₂-adrenergic receptor agonists, serotonin-norepinephrine reuptake inhibitor, a selective serotonin reuptake inhibitor, a selective norepinephrine reuptake inhibitor, a tricyclic antidepressant drug, an α-adrenergic receptor agonist, a female hormone replacement therapy, a surgical therapy, a urinary bladder intima protecting agent, an antiallergic drug, an antihistamine drug, a steroid or bladder distention.

### Brief Description of the Drawings

Fig. 1 shows an effect of the compound A on membrane potential when used alone.
Fig. 2 shows effects of naftopidil on membrane potential when used alone and when used in combination with the compound A.
Fig. 3 shows effects of tamsulosin on membrane potential when used alone and when used in combination with the compound A.

### [Modes for Embodying the Invention]

The present invention is hereinafter described in more detail.

A preparation comprising as an active ingredient the compound represented by the formula (1) or a pharmaceutically acceptable salt thereof, which is an active ingredient of a pharmaceutical composition in the present invention, exhibits an excellent ameliorating effect on lower urinary tract symptoms, not only when used alone, but also when used in combination with an α₁-adrenergic receptor blocker, a 5-α reductase inhibitor, an antiandrogen drug, a β₃-adrenergic receptor agonist, a phosphodiesterase V inhibitor, a plant preparation, an amino acid preparation, a herbal medicine, an intravesical instillation or detrusor injection therapy, a β₂-adrenergic receptor agonist, a serotonin-norepinephrine reuptake inhibitor, a selective serotonin reuptake inhibitor, a selective norepinephrine reuptake inhibitor, a tricyclic antidepressant drug, an α-adrenergic receptor agonist, a female hormone replacement therapy, a surgical therapy, a urinary bladder intima protecting agent, an antiallergic drug, an antihistamine drug, a steroid or bladder distention.

Examples of α₁ receptor blockers include tamsulosin, naftopidil, prazosin, terazosin, urapidil, indoramin, alfuzosin, doxazosin, silodosin, pharmaceutically acceptable salts thereof and the like.
Examples of 5-α reductase inhibitors include finasteride, dutasteride, pharmaceutically acceptable salts thereof and the like.
Examples of antiandrogen drugs include cetrorelix pamoate, ozarelix, teverelix, leuprorelin, chlormadinone acetate, gestonorone caproate, allylestrenol, pharmaceutically acceptable salts thereof and the like.
Examples of selective β₃-adrenergic receptor agonists include mirabegron (YM-178), solabegron (GW-427353), KRP-204, fasobegron, TT-138 (MN-246), KUC-7483, pharmaceutically acceptable salts thereof and the like.
Examples of phosphodiesterase V inhibitors include tadalafil,' vardenafil, sildenafil, UK-369003, pharmaceutically acceptable salts thereof and the like.
Examples of plant preparations include tadenan, permixon, cernilton, prostataplex, eviprostat, pharmaceutically acceptable salts thereof and the like.
Examples of amino acid preparations include paraprost, pharmaceutically acceptable salts thereof and the like.
Examples of herbal medicines include hachimi-jio-gan, gosha-jinki-gan and the like.
Examples of β₂-adrenergic receptor agonists include clenbuterol, pharmaceutically acceptable salts thereof and the like.
Examples of intravesical instillation therapies include intravesical instillation of resiniferatoxin or capsaicin. Examples of detrusor injection therapies include injection of botulinum toxin into the detrusor.
Examples of serotonin-norepinephrine reuptake inhibitors include duloxetine, pharmaceutically acceptable salts thereof and the like.
Examples of selective serotonin reuptake inhibitors include paroxetine, sertraline, fluoxetine, fluvoxamine, pharmaceutically acceptable salts thereof and the like.
Examples of selective norepinephrine reuptake inhibitors include nisoxetine, reboxetine, pharmaceutically acceptable salts thereof and the like.
Examples of tricyclic antidepressant drugs include imipramine, Tryptanol, Anafranil, pharmaceutically acceptable salts thereof and the like.
Examples of α-adrenergic receptor agonists include phenylpropanolamine, ephedrine, methyl ephedrine, midodrine, pharmaceutically acceptable salts thereof and the like.

Examples of surgical treatments such as surgeries for treating benign prostatic hyperplasia include transurethral resection of the prostate (TUR-P), transurethral electrovaporization of the prostate (TUVP), transurethral incision of the prostate (TUI-P), suprapubic prostatectomy, urethral balloon dilation, stenting, laser therapies (VLAP, TULIP, Ho-LEP, ILCP), thermotherapies (transurethral microwave heat treatment (Prostatron, Targis, CoreTherm, TherMatrx), transurethral needle ablation: TUNA), high intensity focused ultrasound (HIFU) and the like.
Examples of female hormones include estrogen and the like. Examples of surgical treatments include sling surgery (tension-free vaginal tape: TVT, transobturator tape: TOT), periurethral collagen (silicon, zuidex) injection, retropubic bladder neck suspension, transvaginal bladder neck suspension, anterior vaginal wall repair, artificial urethral sphincter insertion and the like.
Examples of interstitial cystitis/painful bladder syndrome therapeutic methods include oral ingestion of, or intravesical perfusion therapy with, urinary bladder intima protecting agents such as heparin, dimethylsulfoxide, chondroitin sulfate sodium, pentosan polysulfate, and sodium hyaluronate, ingestion of antiallergic drugs, antihistamine drugs, tricyclic antidepressant drugs or steroids, intravesical instillation therapy with resiniferatoxin, capsaicin, BCG, oxybutynin, or lidocaine, or procedure of bladder distention, and the like.

Examples of pharmaceutically acceptable salts include salts with inorganic acids, such as hydrochlorides, hydrobromides, hydroiodides, sulfates or nitrates, salts with organic acids, for example, acetates, oxalates, citrates, malates, tartrates, fumarates, maleates, methanesulfonates or benzenesulfonates and the like, and the like;
Examples also include salts with organic bases, such as diethanolamine salt, ethylenediamine salt and N-methylglucamine salt, salts with alkaline earth metals, such as calcium salt and magnesium salt, salts with alkali metals, such as lithium salt, potassium salt and sodium salt, and the like.

The above-described compounds may be anhydrides thereof or solvates such as hydrates.

The compound represented by the formula (1) or a pharmaceutically acceptable salt thereof is preferably the 3/2 fumarate of (+)-3-{1-[3-(trifluoromethoxy)benzyl]piperidin-4-yl}-4-phenyl-3,4-dihydro-2(1H)-quinazolinone (hereinafter abbreviated as the compound A) or the like.

A therapeutic agent for lower urinary tract symptom of the present invention comprises a combination of (A) a therapeutic agent for overactive bladder syndrome, a therapeutic agent for pollakiuria/urinary incontinence, a therapeutic agent for benign prostatic hyperplasia, a therapeutic agent for stress urinary incontinence, a therapeutic agent for interstitial cystitis/painful bladder syndrome or the like and (B) the compound A or the like, and may be any agent that allows (A) a therapeutic agent for overactive bladder syndrome, a therapeutic agent for pollakiuria/urinary incontinence, a therapeutic agent for benign prostatic hyperplasia, a therapeutic agent for stress urinary incontinence, a therapeutic agent for interstitial cystitis/painful bladder syndrome or the like and (B) the compound A or the like to be combined at the time of administration. Therefore, provided that it allows (A) a therapeutic agent for overactive bladder syndrome, a therapeutic agent for pollakiuria/urinary incontinence, a therapeutic agent for benign prostatic hyperplasia, a therapeutic agent for stress urinary incontinence, a therapeutic agent for interstitial cystitis/painful bladder syndrome or the like, and (B) the compound A or the like to be combined at the time of administration, the pharmaceutical composition of the present invention may be a single preparation obtained by concurrently formulating (A) a therapeutic agent for overactive bladder syndrome, a therapeutic agent for pollakiuria/urinary incontinence, a therapeutic agent for benign prostatic hyperplasia, a therapeutic agent for stress urinary incontinence, a therapeutic agent for interstitial cystitis/painful bladder syndrome or the like and (B) the compound A or the like, or may be a combination of at least two different preparations obtained by preparing (A) the compound A or the like and (B) a therapeutic agent for overactive bladder syndrome, a therapeutic agent for pollakiuria/urinary incontinence, a therapeutic agent for benign prostatic hyperplasia, a therapeutic agent for stress urinary incontinence, a therapeutic agent for interstitial cystitis/painful bladder syndrome or the like as separate preparations.
The dosage form is not particularly limited; examples include (a) administration as a composition comprising both (A) a therapeutic agent for overactive bladder syndrome, a therapeutic agent for pollakiuria/urinary incontinence, a therapeutic agent for benign prostatic hyperplasia, a therapeutic agent for stress urinary incontinence, a therapeutic agent for interstitial cystitis/painful bladder syndrome or the like and (B) the compound A or the like, that is, as a single preparation, (b) concurrent administration via the same route of administration of two different preparations obtained by preparing (A) a therapeutic agent for overactive bladder syndrome, a therapeutic agent for pollakiuria/urinary incontinence, a therapeutic agent for benign prostatic hyperplasia, a therapeutic agent for stress urinary incontinence, a therapeutic agent for interstitial cystitis/painful bladder syndrome or the like and (B) the compound A or the like as separate preparations, (c) administration via the same route of administration at a time interval of two different preparations obtained by preparing (A) a therapeutic agent for overactive bladder syndrome, therapeutic agent for pollakiuria/urinary incontinence, therapeutic agent for benign prostatic hyperplasia, therapeutic agent for stress urinary incontinence, a therapeutic agent for interstitial cystitis/painful bladder syndrome or the like and (B) the compound A or the like as separate preparations (for example, administration in the order of (A) a therapeutic agent for overactive bladder syndrome, a therapeutic agent for pollakiuria/urinary incontinence, a therapeutic agent for benign prostatic hyperplasia, a therapeutic agent for stress urinary incontinence, a therapeutic agent for interstitial cystitis/painful bladder syndrome or the like and (B) the compound A or the like, or administration in the reverse order), (d) concurrent administration via different routes of administration of two different preparations obtained by preparing (A) a therapeutic agent for overactive bladder syndrome, a therapeutic agent for pollakiuria/urinary incontinence, a therapeutic agent for benign prostatic hyperplasia, a therapeutic agent for stress urinary incontinence, a therapeutic agent for interstitial cystitis/painful bladder syndrome or the like and (B) the compound A or the like as separate preparations, (e) administration via different routes of administration at a time interval of two different preparations obtained by preparing (A) a therapeutic agent for overactive bladder syndrome, a therapeutic agent for pollakiuria/urinary incontinence, a therapeutic agent for benign prostatic hyperplasia, a therapeutic agent for stress urinary incontinence, a therapeutic agent for interstitial cystitis/painful bladder syndrome or the like and (B) the compound A or the like as separate preparations (for example, administration in the order of (A) a therapeutic agent for overactive bladder syndrome, a therapeutic agent for pollakiuria/urinary incontinence, a therapeutic agent for benign prostatic hyperplasia, a therapeutic agent for stress urinary incontinence, a therapeutic agent for interstitial cystitis/painful bladder syndrome or the like and (B) the compound A or the like, or administration in the reverse order) and the like.
In case of administration at a time interval, it is necessary that both (A) a therapeutic agent for overactive bladder syndrome, a therapeutic agent for pollakiuria/urinary incontinence, a therapeutic agent for benign prostatic hyperplasia, a therapeutic agent for stress urinary incontinence, a therapeutic agent for interstitial cystitis/painful bladder syndrome or the like and (B) the compound A or the like be co-present in the body for a time sufficient to ameliorate a lower urinary tract symptom.

The present invention encompasses a commercial package comprising a combination agent consisting of a combination of (A) a therapeutic agent for overactive bladder syndrome, a therapeutic agent for pollakiuria/urinary incontinence, a therapeutic agent for benign prostatic hyperplasia, a therapeutic agent for stress urinary incontinence, a therapeutic agent for interstitial cystitis/painful bladder syndrome or the like and (B) the compound A or the like, and a printed matter concerning the combination agent stating that the combination agent can be used, or should be used, for the treatment of lower urinary tract symptoms; a commercial package comprising a pharmaceutical composition comprising a therapeutic agent for overactive bladder syndrome, a therapeutic agent for pollakiuria/urinary incontinence, a therapeutic agent for benign prostatic hyperplasia, a therapeutic agent for stress urinary incontinence, a therapeutic agent for interstitial cystitis/painful bladder syndrome or the like, and a printed matter concerning the pharmaceutical composition stating that the pharmaceutical composition can be used, or should be used, for the treatment of lower urinary tract symptoms with the compound represented by the formula (1) or the like; and a commercial package comprising (A) a pharmaceutical composition comprising the compound A or the like and (B) a printed matter concerning the pharmaceutical composition stating that the pharmaceutical composition can be used, or should be used, for the treatment of lower urinary tract symptoms.

A therapeutic agent for lower urinary tract symptom of the present invention is useful in the treatment of lower urinary tract symptoms manifested in overactive bladder syndrome, benign prostatic hyperplasia, mixed urinary incontinence or interstitial cystitis/painful bladder syndrome and the like.

A therapeutic agent for lower urinary tract symptom of the present invention, when used for treatment, can be administered as a pharmaceutical composition orally or non-orally (for example, intravenous, subcutaneous, or intramuscular injection, topical, transrectal, transdermal, or transnasal). Examples of compositions for oral administration include tablets, capsules, pills, granules, powders, solutions, suspensions and the like; examples of compositions for non-oral administration include aqueous or oily agents for injection, ointments, creams, lotions, aerosols, suppositories, plasters and the like. These preparations are prepared using conventionally known techniques, and can contain a non-toxic, inert carrier or additive in common use in the pharmaceutical field.

The dose of a therapeutic agent for lower urinary tract symptom of the present invention varies depending on the compound, as well as on the patient disease, age, body weight, sex, symptoms, route of administration and the like; usually, for an adult (weight 50 kg), each of a therapeutic agent for overactive bladder syndrome, a therapeutic agent for pollakiuria/urinary incontinence, a therapeutic agent for benign prostatic hyperplasia, a therapeutic agent for stress urinary incontinence or a therapeutic agent for interstitial cystitis/painful bladder syndrome, and the compound A, is administered at 0.01 to 3000 mg/day, preferably 0.1 to 2550 mg/day, once or in two or three divided doses a day. The same can also be administered once per several days to several weeks.

### Examples

The present invention is hereinafter described more specifically by means of the following reference examples, working examples and test examples, to which, however, the present invention is not limited. The compound names shown in the following reference examples and working examples do not always conform to the IUPAC Nomenclature. While abbreviations are sometimes used for the sake of simplifying the description, these abbreviations have the same definitions as those given above.

### Examples

### Test Example 1: Action of the compound A on C-fiber activation

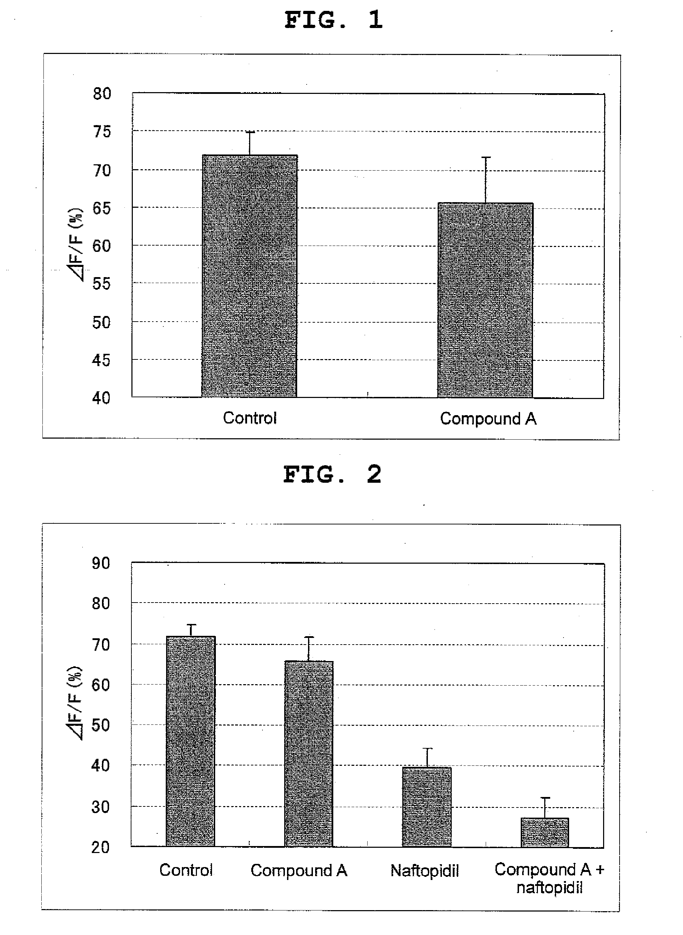

This test was performed using male SD strain rats at 6 to 7 weeks of age after birth. The animals were reared in metal cages, and allowed to take food (CE-2; CLEA Japan) and water ad libitum.
Rat spinal cord was dissected under halothane anesthesia. DRG tissue was removed from the spinal cord under a stereoscopic microscope, and recovered into an ice-cooled culture dish containing DMEM. The DRG tissue was digested using 0.25% trypsin and 0.38% collagenase, and DRG neurons were isolated. The DRG neurons were seeded to a cover glass coated with Poly-L-Lysine and laminin, and cultured.
On the day after the start of cultivation, the DRG neurons were loaded with 5 µM DiBAC₄ (3) in the presence of 5% gaseous carbon dioxide at 37°C for 1 hour or more. Thereafter, the neurons were transferred to an assay buffer solution (pH 7.3; 10 mM HEPES, 145 mM NaCl, 5 mM KCl, 2 mM CaCl₂, 1 mM MgCl₂, 10 mM Glucose), and the fluorescence intensity of each DRG neuron was measured using the image analytical system AQUACOSMOS (Hamamatsu Photonics).
When small DRG neurons were selected and stimulated with 60 mM high-concentration K solution (pH 7.3; 10 mM HEPES, 90 mM NaCl, 60 mM KCl, 2 mM CaCl₂, 1 mM MgCl₂, 10 mM Glucose), a rise in membrane potential was observed. When the 60 mM high-concentration K solution was replaced with the assay buffer solution, the transiently-elevated membrane potential fell rapidly. When the same was again stimulated with the 60 mM high-concentration K solution, a rise in membrane potential was observed with high reproducibility. Compared to the first rise in membrane potential (ΔF/F) as 100%, the ratio of the second rise in membrane potential was 72±3% (control). Compound A (3 µM) was pretreated between the 1st and 2nd stimulations.
The effect of the compound A on the rise in membrane potential when used alone is shown in Fig. 1. With the pretreatment with the compound A (3 µM), the rise in membrane potential (ΔF/F) was suppressed to 66±6%.
It is seen that the compound A has a suppressing effect on C-fiber activation when used alone.

### Test Example 2: Combination effect with α₁-adrenergic receptor blocker

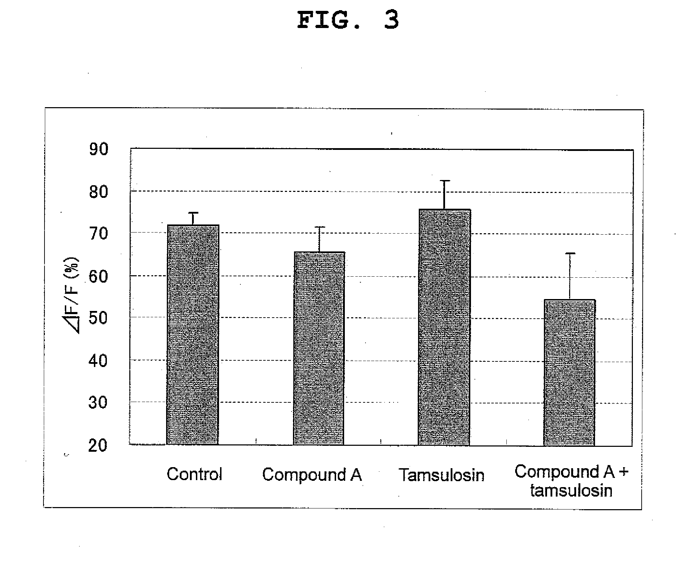

This test was performed using male SD strain rats at 6 to 7 weeks of age after birth. The animals were reared in metal cages, and allowed to take food (CE-2; CLEA Japan) and water ad libitum.
Rat spinal cord was dissected under halothane anesthesia. DRG tissue was removed from the spinal cord under a stereoscopic microscope, and recovered into an ice-cooled culture dish containing DMEM. The DRG tissue was digested using 0.25% trypsin and 0.38% collagenase, and DRG neurons were isolated. The DRG neurons were seeded to a cover glass coated with Poly-L-Lysine and laminin, and cultured.
On the day after the start of cultivation, the DRG neurons were loaded with 5 µM DiBAC₄ (3) in the presence of 5% gaseous carbon dioxide at 37°C for 1 hour or more. Thereafter, the neurons were transferred to an assay buffer solution (pH 7.3; 10 mM HEPES, 145 mM NaCl, 5 mM KCl, 2 mM CaCl₂, 1 mM MgCl₂, 10 mM Glucose), and the fluorescence intensity of each DRG neuron was measured using the image analytical system AQUACOSMOS (Hamamatsu Photonics).
When small DRG neurons were selected and stimulated with 60 mM high-concentration K solution (pH 7.3; 10 mM HEPES, 90 mM NaCl, 60 mM KCl, 2 mM CaCl₂, 1 mM MgCl₂, 10 mM Glucose), a rise in membrane potential was observed. When the 60 mM high-concentration K solution was replaced with the assay buffer solution, the transiently-elevated membrane potential fell rapidly. When the same was again stimulated with the 60 mM high-concentration K solution, a rise in membrane potential was observed with high reproducibility. Compared to the first rise in membrane potential (ΔF/F) as 100%, the ratio of the second rise in membrane potential was 72±3% (control). The drug was treated between the 1st and 2nd stimulations.
The effects of naftopidil on the rise in membrane potential when used alone and when used in combination with the compound A are shown in Fig. 2. With the pretreatment with naftopidil (30 µM), the rise in membrane potential (ΔF/F) was suppressed to 40±5%. When the compound A (3 µM) and naftopidil (30 µM) were used in combination, the rise in membrane potential (ΔF/F) was suppressed to 27±5%.
Next, the effects of tamsulosin when used alone and when used in combination with the compound A are shown in Fig. 3. The change in membrane potential due to the pretreatment with tamsulosin (0.1 µM) was to nearly the same extent as with the control. When the compound A (3 µM) and tamsulosin (0.1 µM) were used in combination, the rise in membrane potential (ΔF/F) was suppressed to 55±11.
It is evident from Fig. 2 that the compound A and naftopidil have a suppressing action on the high-concentration K-induced rise in membrane potential, that is, C-fiber activation-suppressing action. Furthermore, it is evident from Fig. 2 and Fig. 3 that the compound A has a combination effect with naftopidil and tamsulosin.

### Test Example 3: Combination effect with β₃-adrenergic receptor agonist

This test is performed using male SD strain rats at 8 to 10 weeks of age after birth. The animals are reared in metal cages, and allowed to take food (CE-2; CLEA Japan) and water ad libitum.
While each rat is under halothane anesthesia, a cannula for measuring inner pressure is inserted into the urinary bladder. Four days after placement of the cannula, a cannula for drug administration is inserted into the stomach while the animal is under halothane anesthesia. The following day, while the animal is awake, physiological saline is perfused through the urinary bladder via a urinary bladder cannula at a rate of 6 mL/h for 1 hour or more. After the voiding interval has stabilized, the physiological saline is replaced with physiological saline containing 10 µM PGE₂, which is perfused for 1 hour or more. After the voiding interval has shortened and stabilized, bladder capacity is measured. Bladder capacity is calculated as the sum of voided volume and residual urine volume. Compound A and YM-178:

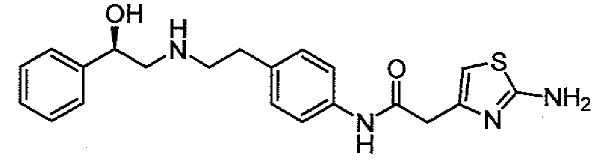

or a mixed liquid thereof is administered via an intragastric cannula. Bladder capacity is measured until 90 minutes after the administration. The bladder capacity is compared between before and after the administration.
The bladder capacity increasing action is enhanced when the compound A and YM-178 are used in combination, than when each is administered alone.

### Industrial Applicability

The present invention makes it possible to provide a novel useful therapeutic agent for lower urinary tract symptoms.

## Claims

1. A therapeutic agent for lower urinary tract symptom comprising (+)-3-{1-[3-(trifluoromethoxy)benzyl]piperidin-4-yl}-4-phenyl-3,4-dihydro-2(1H)-quinazolinone or a pharmaceutically acceptable salt thereof as an active ingredient.

2. A therapeutic agent for lower urinary tract symptom comprising a combination of (+)-3-{1-[3-(trifluoromethoxy)benzyl]piperidin-4-yl}-4-phenyl-3,4-dihydro-2(1H)-quinazolinone or a pharmaceutically acceptable salt thereof, and a drug selected from the group consisting of a therapeutic agent for overactive bladder syndrome, a therapeutic agent for pollakiuria/urinary incontinence, a therapeutic agent for benign prostatic hyperplasia, a therapeutic agent for stress urinary incontinence and a therapeutic agent for interstitial cystitis/painful bladder syndrome.

3. A therapeutic agent for lower urinary tract symptom comprising a combination of (+)-3-{1-[3-(trifluoromethoxy)benzyl]piperidin-4-yl}-4-phenyl-3,4-dihydro-2(1H)-quinazolinone or a pharmaceutically acceptable salt thereof, and a drug selected from the group consisting of an α₁-adrenergic receptor blocker, a 5-α reductase inhibitor, an antiandrogen drug, a β₃-adrenergic receptor agonist, a phosphodiesterase V inhibitor, a plant preparation, an amino acid preparation, a herbal medicine, an intravesical instillation or detrusor injection therapy, a β₂-adrenergic receptor agonist, a serotonin-norepinephrine reuptake inhibitor, a selective serotonin reuptake inhibitor, a selective norepinephrine reuptake inhibitor, a tricyclic antidepressant drug, an α-adrenergic receptor agonist, a female hormone, a urinary bladder intima protecting agent, an antiallergic drug, an antihistamine drug and a steroid.

4. The therapeutic agent for lower urinary tract symptom according to claim 2, wherein the therapeutic agent for benign prostatic hyperplasia is an α₁-adrenergic receptor blocker.

5. The therapeutic agent for lower urinary tract symptom according to claim 4, wherein the α₁-adrenergic receptor blocker is selected from the group consisting of naftopidil and tamsulosin.

6. The therapeutic agent for lower urinary tract symptom according to claim 2, wherein the therapeutic agent for benign prostatic hyperplasia is a 5-α reductase inhibitor.

7. The therapeutic agent for lower urinary tract symptom according to claim 2, wherein the therapeutic agent for benign prostatic hyperplasia is an antiandrogen drug.

8. The therapeutic agent for lower urinary tract symptom according to claim 2, wherein the therapeutic agent for overactive bladder syndrome is a β-adrenergic receptor agonist.

9. The therapeutic agent for lower urinary tract symptom according to claim 8, wherein the β-adrenergic receptor agonist is a selective β₃-adrenergic receptor agonist.

10. The therapeutic agent for lower urinary tract symptom according to claim 2, wherein the therapeutic agent for overactive bladder syndrome or the therapeutic agent for benign prostatic hyperplasia is a phosphodiesterase V inhibitor.

11. The therapeutic agent for lower urinary tract symptom according to claim 2, wherein the therapeutic agent for benign prostatic hyperplasia is a plant preparation, an amino acid preparation, or a herbal medicine.

12. A combination therapy with (+)-3-{1-[3-(trifluoromethoxy)benzyl]piperidin-4-yl}-4-phenyl-3,4-dihydro-2(1H)-quinazolinone or a pharmaceutically acceptable salt thereof, and intravesical instillation of resiniferatoxin or capsaicin or detrusor injection of botulinum toxin, for treating lower urinary tract symptoms.

13. The therapeutic agent for lower urinary tract symptom according to claim 2, wherein the therapeutic agent for stress urinary incontinence is a β₂-adrenergic receptor agonist.

14. The therapeutic agent for lower urinary tract symptom according to claim 2, wherein the therapeutic agent for stress urinary incontinence is a serotonin-norepinephrine reuptake inhibitor, a selective serotonin reuptake inhibitor, or a selective norepinephrine reuptake inhibitor.

15. The therapeutic agent for lower urinary tract symptom according to claim 2, wherein the therapeutic agent for stress urinary incontinence or the therapeutic agent for pollakiuria/urinary incontinence is a tricyclic antidepressant drug.

16. The therapeutic agent for lower urinary tract symptom according to claim 2, wherein the therapeutic agent for stress urinary incontinence is an α-adrenergic receptor agonist.

17. A combination therapy with (+)-3-{1-[3-(trifluoromethoxy)benzyl]piperidin-4-yl}-4-phenyl-3,4-dihydro-2(1H)-quinazolinone or a pharmaceutically acceptable salt thereof, and a surgery for treating benign prostatic hyperplasia, for treating lower urinary tract symptoms.

18. A combination therapy with (+)-3-{1-[3-(trifluoromethoxy)benzyl]piperidin-4-yl}-4-phenyl-3,4-dihydro-2(1H)-quinazolinone or a pharmaceutically acceptable salt thereof, and a female hormone replacement therapy, for treating lower urinary tract symptoms.

19. A combination therapy with (+)-3-{1-[3-(trifluoromethoxy)benzyl]piperidin-4-yl}-4-phenyl-3,4-dihydro-2(1H)-quinazolinone or a pharmaceutically acceptable salt thereof, and a surgery for the treatment of stress urinary incontinence, for treating lower urinary tract symptoms.

20. A combination therapy with (+)-3-{1-[3-(trifluoromethoxy)benzyl]piperidin-4-yl}-4-phenyl-3,4-dihydro-2(1H)-quinazolinone or a pharmaceutically acceptable salt thereof, and an interstitial cystitis/painful bladder syndrome therapy, for treating lower urinary tract symptoms.
